# EUROPEAN PATENT APPLICATION

(11) **EP 4 170 012 A2**
(43) Date of publication of application: **26.04.2023**
(21) Application number: 22210557.9
(22) Date of filing: 11.06.2021
(51) Int. Cl.: C12M 1/42, C12M 1/00, B01L 3/00, B01L 9/06

(54) **CLOSED FLUID RECEIVING AND SAMPLING CONTAINER**

(30) Priority: 16.06.2020 US 202016903270; 23.11.2020 US 202017102142; 29.04.2021 US 202117244373
(62) Divisional of application: 21825858.0
(71) Applicant: SaniSure, Inc., Camarillo, CA 93012 (US)
(72) Inventor: BALLEW, Chris, Thousand Oaks (US); SHOR, Richard, Moorpark (US)
(74) Representative: Patentanwälte Olbricht Buchhold Keulertz

(57) **Abstract**

A closed fluid receiving and sampling container that enables transfer of valuable reaction liquid to a receptacle without risking loss of sterility. The sampling container has a dip tube subassembly with a shorter inlet tube bent towards the wall of the receptacle to prevent or reduce foaming, and a longer outlet tube used to drain the waste liquid once the magnetic beads are trapped by the magnet. The dip tube subassembly is injection molded in one piece and provides a sealed cap also with a vent tube therethrough to enable filling and draining the receptacle without removing the cap, thus keeping the process aseptic. The sampling container is especially useful in the context of magnetic bead separation processes.

## Description

### RELATED APPLICATION INFORMATION

This application is a PCT of U.S. Patent Application Serial No. 17/244,373, filed April 29, 2021, which is a continuation-in-part of U.S. Patent Application Serial No. 17/102,142, filed November 23, 2020, which is a continuation of U.S. Patent Application Serial No. 16/903,270, filed June 16, 2020, now Patent No. 10,843,186, entitled CLOSED FLUID RECEIVING AND SAMPLING CONTAINER the disclosure of which is hereby incorporated by reference in its entirety.

### BACKGROUND

### Field

This disclosure relates to a closed system for receiving liquid chemical or biological reaction products that facilitates sampling.

### Description of the Related Art

Various chemical and biological reactions generate liquid reaction products that are often quite valuable due to a number of factors, including the fragile nature of the reaction and limited yield. Recovering the target product from the surrounding media can be difficult.

For instance, bioreactors that generate stem cells often require multiple stages and yield a relatively small amount of usable cells. To separate the cells from the surrounding media, various techniques are known, including bead separation. Bead separation relies on the capacity of small beads to bond to the substance of interest, which locates that substance on the beads for easier retrieval.

The Invitrogen division of ThermoFisher Scientific based in Carlsbad, CA offers the Dynabeads magnetic separation technology for this
purpose (https://www.thermofisher.com/us/en/home/brands/product-brand/dynal.html). The liquid product from a reaction is ported to a container such as a test tube which contains small beads. The beads are magnetically attractive and are coated so as to bind to the target material, e.g., stem cells, T-cells, nucleic acids, proteins, etc. The container is placed in a housing having a magnetic field along one side so that the beads collect in one area. The remaining media can then be drained away, leaving the valuable cells adhered to the beads. The cells may be used still attached to the
beads, or the cells are washed from the beads with an inert solution and then recovered. Bead separation techniques may also be used for virus production, bacteria, chemical or small molecule chemical processes, and large molecule processes (such as when differentiated from proteins like organelles).

The process described above requires transport of the cell culture from the reactor to the microbead container, which can introduce contamination. There remains a need for a way to transfer valuable reaction liquid to a separation container without risking loss of sterility. Other processes not requiring bead separation may also benefit from a sterile transfer system.

### SUMMARY OF THE INVENTION

The present application discloses a sampling container that enables transfer of valuable reaction liquid to a separation container without risking loss of sterility. The sampling container is especially useful in the context of magnetic bead separation processes.

The sampling container has a dip tube subassembly with a shorter inlet tube bent towards the wall of the container to prevent or reduce foaming, and a longer outlet tube used to drain the waste liquid once the magnetic beads are trapped by the magnet. This allows for the introduction of product through the inlet tube and withdrawal of all of the waste liquid through the outlet tube which goes all the way to the bottom for the removal of every last drop. The dip tube subassembly has a sealed cap also with a vent tube therethrough which provides the ability to fill and drain the container without removing the cap, thus keeping the process aseptic. The sampling container disclosed herein essentially provides a closed system with built-in pipettes. The container has a sealed cap through which the pipettes and a vent tube pass. The dip tube subassembly is injection molded and provides a sealed barrier lid that incorporates the vent and pipette tubes.

In one embodiment disclosed herein, a closed system for receiving and sampling liquid, comprises a receptacle with solid walls and no openings defining a closed bottom end and an open top end, the closed bottom end being narrowed and not flat. Preferably, the receptacle has an inner volume of 50 ml or less, though volumes up to 1 liter are contemplated. A subassembly is sealed over the open top end of the receptacle to close the inner volume. The subassembly has a lid, a first tube and first connector, a second tube and second connector, and a vent connector. Preferably, the subassembly is injection molded as one piece. The first and second tubes extend downward from the lid and are each, respectively, in fluid communication with the first and second connectors projecting upward from the lid. A vent connector projecting upward from the lid opens to a lower side of the lid. When the lid is sealed over the top end of the receptacle the first, second and vent connectors provide exclusive avenues of ingress and egress to and from the inner volum. Finally, a ring-shaped cap secures around the open top end of the receptacle so as to retain the lid over the open top end, wherein the closed system is manufactured and sold in a closed configuration with the first, second and vent connectors being sealed. The second tube preferably extends farther downward into the inner volume than the first tube.

In one embodiment, the first tube extends vertically downward and curves radially outward at a lower end, and is positioned to be spaced closely adjacent to an inner wall of the closed bottom end of the receptacle. For instance, the lower end curves 90° or less and an end face of the lower end is angled perpendicular to the inner wall of the closed bottom end of the receptacle.

In another embodiment, receptacle may narrow inward at the closed bottom end to a central location, and the lower end of the second tube angles so as to terminate in close proximity to the central location of the closed bottom end. For example, second tube terminates within 0.05 inches of the closed bottom end of the receptacle. Also, the second tube may have a small cut out across its longitudinal axis at its lower end to prevent the lower end from sealing against the closed bottom end of the receptacle from suction.

A flexible first tube may be connected to the first connector and a first plug provided for sealing the flexible first tube, and a flexible second tube may be connected to the second connector and a second plug provided for sealing the flexible second tube. In addition, an on/off pinch valve may be provided on both the flexible first tube and the flexible second tube to enable manual opening and closure of flow therethrough. The closed system may further include a vent filter connected to the vent connector.

### DESCRIPTION OF THE DRAWINGS

FIG. 1A is an elevational view of an exemplary fluid receiving and sampling container along with complementary fluid connections, and FIG. 1B shows the container enlarged;
FIGS. 2A is a perspective view of an input/output insert for the fluid receiving and sampling container, and FIGS. 2B-2D are orthogonal views thereof; and
FIG. 3 shows an exemplary system incorporating the fluid receiving and sampling container which uses magnetic beads to separate usable cells from surrounding media.
FIG. 4 shows another exemplary system incorporating the fluid receiving and sampling container to recirculate medium from a bioreactor.
FIG. 5 illustrates a system having multiple fluid receiving and sampling containers connected in series and to a bioreactor.
FIG. 6 is a schematic diagram of a centrifuge for processing a plurality of fluid receiving and sampling containers at once.

### DETAILED DESCRIPTION

The present application discloses a sealed container for receiving and sampling fluid without opening the container. The container may be provided in a variety of sizes, including small flasks or test tubes that are useful in magnetic bead separation technology. Although the sealed container is especially useful with bead separation technology, other applications are contemplated and the system should not be considered limited to any particular usage.

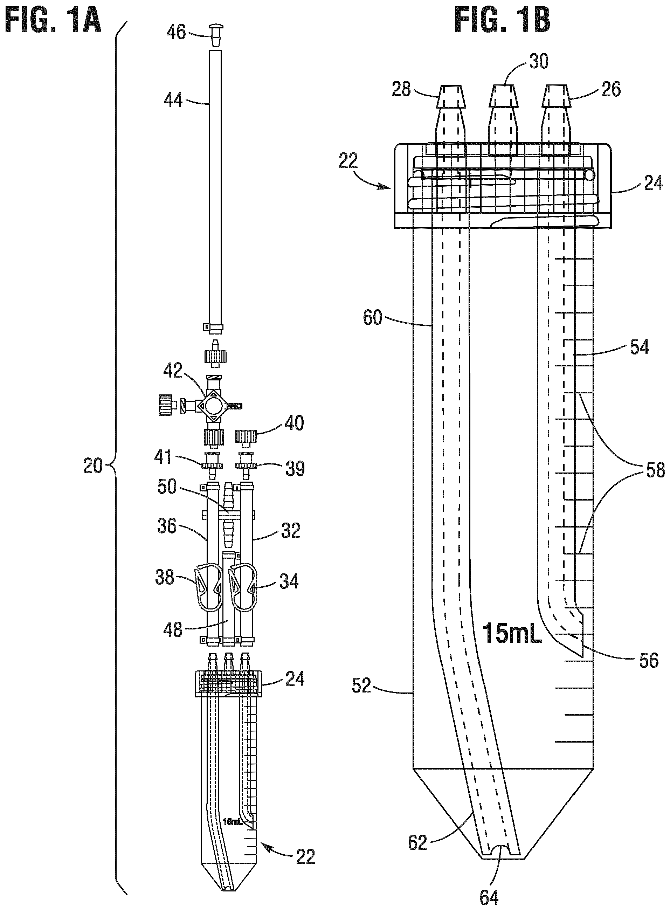

FIG. 1A is an elevational view of an exemplary system 20 including a sealed fluid receiving and sampling container 22 along with complementary fluid connections, and FIG. 1B shows the container enlarged. The container 22 has a ring-shaped cap 24, preferably screwed on, securing at least three upstanding connectors. In particular, an inlet connector 26, an outlet connector 28, and a vent connector 30 are shown in FIG. 1B. The connectors 26, 28, 30 may be barbed fittings or other such configurations for mating with flexible tubes, or other known fluid connectors.

FIG. 1A shows an assembly of tubes and associated hardware to provide communication with the interior of the container 22. Namely, a first flexible tube 32 having an on/off pinch valve 34 may be connected to the inlet connector 26, and a second flexible tube 36 having an on/off pinch valve 38 connects to the outlet connector 28. An intermediate fitting 39 may be provided at an outer end of the first flexible tube 32 for mating with a source fitting (not shown), and a cap or plug 40 can be used to seal the intermediate fitting. A similar intermediate fitting 41 can be attached to the outer end of the second flexible tube 36 for mating with an outlet fitting, such as provided on a three-way stopcock 42. From there, the junctions on the stopcock 42 enable distribution of the fluid from the outlet tube 60 in several directions, such as through a third flexible tube 44 having a removable plug 46 on its distal end.

A vent tube 48 leads from the vent connector 30 to a disc filter 50. The atmosphere within the container 22 may require special filtering to avoid toxicity or other contamination of the laboratory environment.

The sealed fluid receiving and sampling container 22 is shown enlarged in FIG. 1B, and includes a closed bottom receptacle 52 having an open upper end to which the cap 24 attaches. The receptacle 52 has solid side and bottom walls without openings so that the only ingress or egress is through the open upper end.

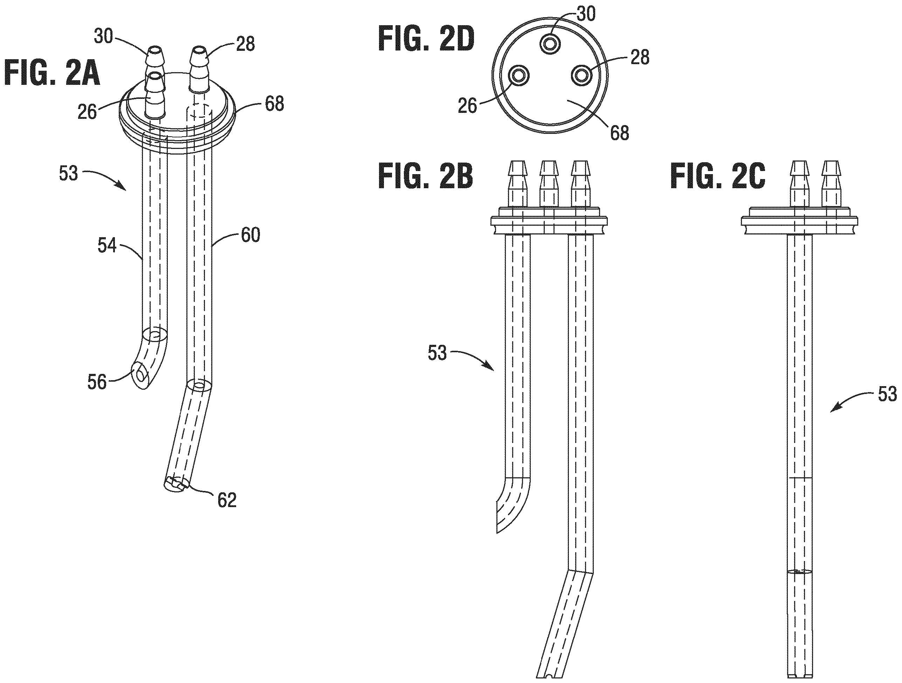

An insert subassembly 53 shown in FIGS. 2A-2D is retained within an interior cavity of the receptacle 52 by the ring-shaped cap 24. The insert subassembly 53 comprises an inlet tube 54 leading downward from the inlet connector 26 to a curved lower end 56. The lower end 56 preferably curves 90° or less radially outward and terminates closely adjacent an inner wall of the receptacle 52 to reduce foaming of any fluid entering the container. More specifically, an end face of the lower end 56 is desirably angled perpendicular to the adjacent inner wall of the receptacle
52. Thus, if the innerwall of the receptacle 52 is vertical, as shown, the lower end 56 curves90°.
However, for receptacles with curved or angled walls, the lower end 56 curves less than 90°, such as 63°, down to an angle as small as 20°.

Volumetric index marks 58 are desirably provided on the exterior of the receptacle 52. In a preferred embodiment, the curved lower end 56 curves radially outward into close proximity with an inner wall of the receptacle 52 at a 15 mL index mark. In one embodiment, the curved lower end 56 is spaced as close as 0.1 inches or less from the inner wall of the receptacle 52. The receptacle 52 preferably has a useful volume of 50 ml, but smaller receptacles down to 5 or 15 ml are contemplated, and medium-sized ones up to 100 ml or even 500 ml, as well. Smaller volume receptacles 52 (up to 500 ml) with narrowed (conical, dished or rounded) bottom ends are useful as they can then be transferred to a centrifuge for concentrating solids at the bottom.

The insert subassembly 53 also includes a siphon or outlet tube 60 leading downward from the outlet connector 28 to an angled siphon end 62 in close proximity to a closed bottom end of the receptacle 52. The closed bottom end of the receptacle 52 may be tapered so that the siphon end 62 is capable of removing all but trace amounts of liquid within the receptacle. As the outlet tube 60 is not centered in the lid 68, the siphon end 62 may be slightly angled relative to a longitudinal axis of the receptacle 52 to reach the tapered bottom end. Further, the lower siphon end 62 may have a small cut out 64 formed perpendicularly across its longitudinal axis which helps prevent the siphon end 62 from sealing against the closed bottom end of the receptacle 52 due to suction. In one embodiment, the siphon end 62 reaches to within 0.05 inches of the bottom of the receptacle 52, and preferably about 0.03 inches.

It should be noted here that the function of the tubes 54, 60 may be reversed, with the former being used for an outlet and the latter for an inlet. Both tubes 54, 60 terminate in open distal ends 56, 64, respectively, that reduce foaming if used as an inlet. That is, the ends 56, 64 open close to the interior wall of the receptacle 52 so that fluid ported in will not splash or otherwise be inordinately churned. Although the longer tube 60 is advantageous for siphoning fluids and matter from the very bottom of the receptacle 52, some processes may require removal of the solution or colloidal solution above detritus at the bottom, which makes the shorter tube 54 the better choice.

The three connectors 26, 28, 30 and tubes 54, 60 are fixed with respect to a disk - shaped lid 68. The lid 68 fits closely within an upper end of the receptacle 52 and is sealed therein by the cap 24. The lid 68 may have an elastomeric outer periphery (e.g., O-ring) to provide a fluid seal against an inner surface of the receptacle 52. In this way, the only three avenues of fluid communication between the internal cavity of the receptacle 52 and the outside is through the three connectors 26, 28, 30. Fluid enters the receptacle 52 through the inlet connector 26 and inlet tube 54, and may be withdrawn through the outlet tube 60 and outlet connector 28. Any excess pressure created by the fluid transfer may result in air or other gas venting through the vent connector30.

The insert subassembly 53 is desirably injection molded in one piece of a suitable polymer, such as polypropylene. The term "injection molded in one piece" means that the subassembly 53 is formed in a single manufacturing operation from a homogenous polymer so that there are no separable parts. This not only creates efficiencies of fabrication but reduces the potential for contamination during assembly with the receptacle 52 and cap 24, as well as during integration with a larger assembly and during use. In an exemplary embodiment, the receptacle 52 is also polypropylene and has a capacity of 50 mL, but sizes as large as 500 ml or even 1 liter are contemplated. The vertical height of the smaller 50 ml insert subassembly 53 may be around 5 inches, with the height of the receptacle 52 being slightly less. The inlet and outlet tubes 54, 60, as well as the vent connector 30 may have a variety of inner diameters, such as around 0.1 inches. Of course, these dimensions are exemplary and can be modified and scaled up for largersystems.

Once the insert subassembly 53, and specifically the lid 68, is placed over the open upper end of the receptacle 52 and is sealed therein by the 24, the interior of the container 22 is sealed and the only avenues to introduce or remove material are through the three connectors 26, 28, 30.

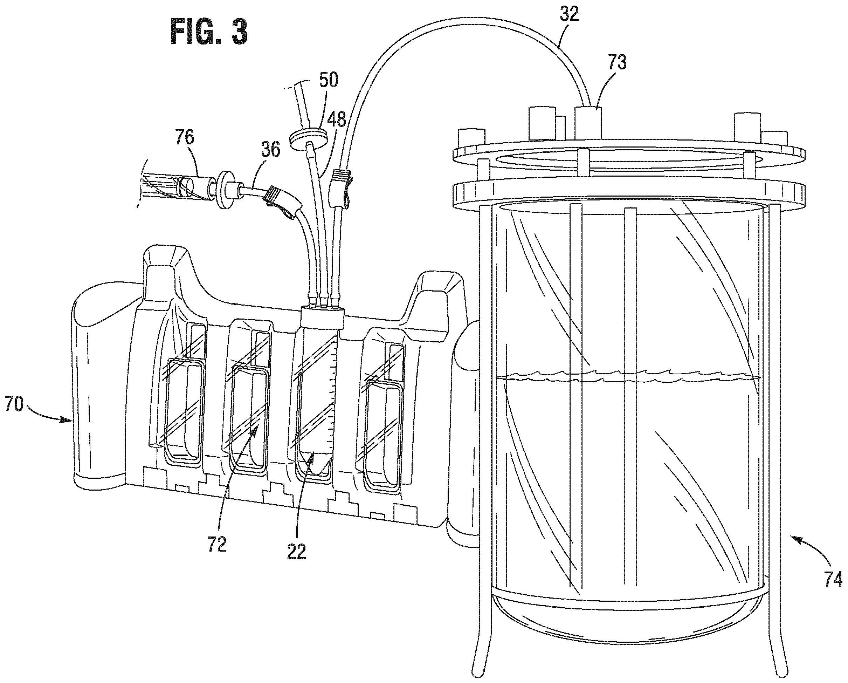

FIG. 3 shows an exemplary system incorporating the fluid receiving and sampling container 22. In this context, the container 22 is held vertically by a stand 70 having a plurality of stations 72 adapted for holding the container. The first flexible tube 32 that attaches to the inlet connector 26 extends to an outlet 73 of a source of fluid such as a bioreactor 74. The source of fluid may be a chemical or biological reactor, a larger fluid receptacle, or a flask similar to the container 22 described herein. The second flexible tube 36 in fluid communication with the outlet connector 28 extends to a receiving container such as a syringe 76.

The entire system 20 such as shown in FIG. 1A (or just the container 22 in FIG. 1B) may be sold as a closed, sterile assembly in suitable sterile packaging. A user need only remove the various plugs and attached the inlet and outlet connectors to appropriate source and receiving containers to effectuate retrieval and sampling of the desirable liquid. The system 20 remains closed during the entire process, which greatly reduces the chance of any contamination, thus increasing aggregate yield of the final product.

The closed sterile container 22 in FIG. 1B provides a convenient vehicle for speeding up many chemical and biological processes. The container 22 is sold in a closed, sterile condition suitable for hooking up right out of the shipping container by the customer to a larger system, such as those shown herein. That is, the closed system 20 or container 22 is manufactured and sold in a sterile configuration, with the first, second and vent connectors 26, 28, 30 being plugged directly or via a plug on an attached flexible tube. By simply removing a sterile cap on one or more of the connectors 26, 28, 30, or from a tube attached thereto, and making the appropriate connections, the container 22 may be integrated into a larger reactor system to perform selected tasks. In its most basic form the container 22 provides a convenient concentrator for the larger reactor which may be used in a variety of ways, from those as prosaic as removing desirable or undesirable cells, to those which accelerate and work in conjunction with the main reaction. While the main reaction such as occurring in the bioreactor 74 continues, samples of reactor solution may be removed, treated in the container 22, and then re-introduced into the larger reactor, all without opening any lids or ports or otherwise exposing the reaction contents to contamination.

A useful application for the seal receiving and sampling container 22 uses magnetic beads to separate usable cells from surrounding media. More particularly, small magnetic beads are coated with a material which attracts desirable (or undesirable) cells. The stand 70 has magnets or is magnetized on its inner vertical wall at each of the stations 72 which attracts the magnetic beads suspended in fluid within the container 22 and holds them against the inside wall of the container. With the magnetic beads immobilized in this manner, any residual chemical media may be withdrawn through the outlet tube 60 using the syringe 76, for example, and discarded or otherwise utilized if desired. Subsequently, additional washing fluid may be introduced to the container 22 to separate the desirable cells from the magnetic beads. By placing the container 22 once more in the stand 70, the cleaned magnetic beads maybe once again immobilized so that the cells within the wash fluid can be removed through the outlet tube 60 and exterior tube 36. In some cases, the beads are calibrated to attract undesirable matter, in which case it is the contents of the remaining solution that is useful. In such a situation, the valuable solution may be drained from the container 22 while the magnetic beads are immobilized.

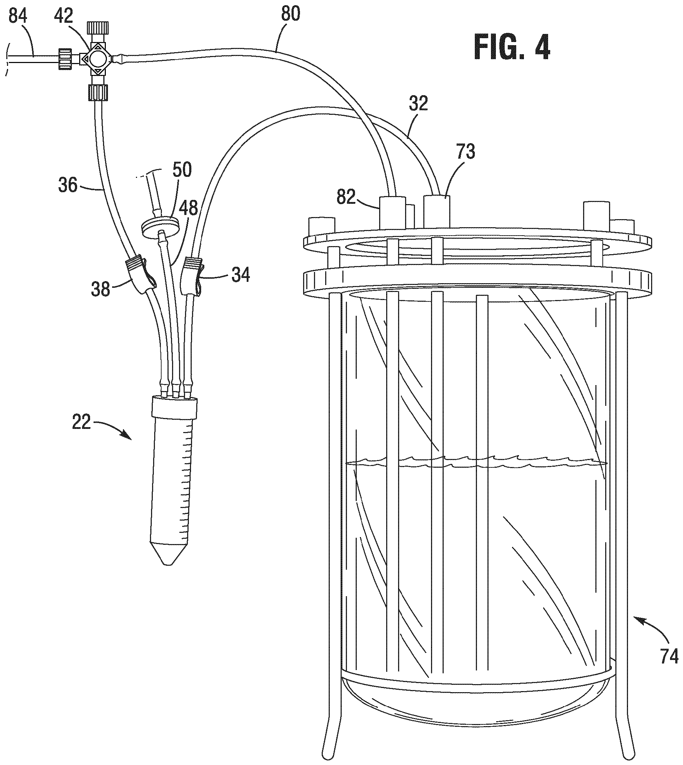

FIG. 4 shows another exemplary system incorporating the fluid receiving and sampling container 22 to recirculate medium from a bioreactor 74. Some cell therapy applications utilize medium recirculation to enhance the equality of cells in growth phase, expansion and differentiation. Medium may be ported through the bioreactor outlet 73 to the container 22, and then through the flexible tube 36 and back via the stopcock 42 and return passage or tube 80 into a bioreactor inlet 82. Recirculation of medium through container 22 has the ability to remove waste while allowing the cells to either remain in the bioreactor and/or pass through the enclosed loop without exposing cells to risk of contamination. In addition, the stopcock 42 may be used to alter the flow path of the recirculating medium to a sampling or diverter tube 84 to be redirected into a subsequent processing container (not shown). Again, the sealed nature of the system 20 including the fluid receiving and sampling container 22 provides a "plug-and-play" assembly which may be readily connected to the bioreactor 74 to supply a recirculation loop without risk of contamination.

Another benefit of connecting the closed-loop fluid receiving and sampling container 22 to the bioreactor 74 is being able to intensify the main reaction. That is, cells of interest may be removed and concentrated within the container 22, enabling isolated treatment with added nutrients to encourage cell growth and/or differentiation. Once the isolated growth phase has taken place in the smaller container 22, the products, perhaps minus waste components, are then ported back into the bioreactor 74. This enables a more concentrated or efficient growth to take place outside of the larger bioreactor 74, which then charges the bioreactor for subsequent cell expansion and/or differentiation.

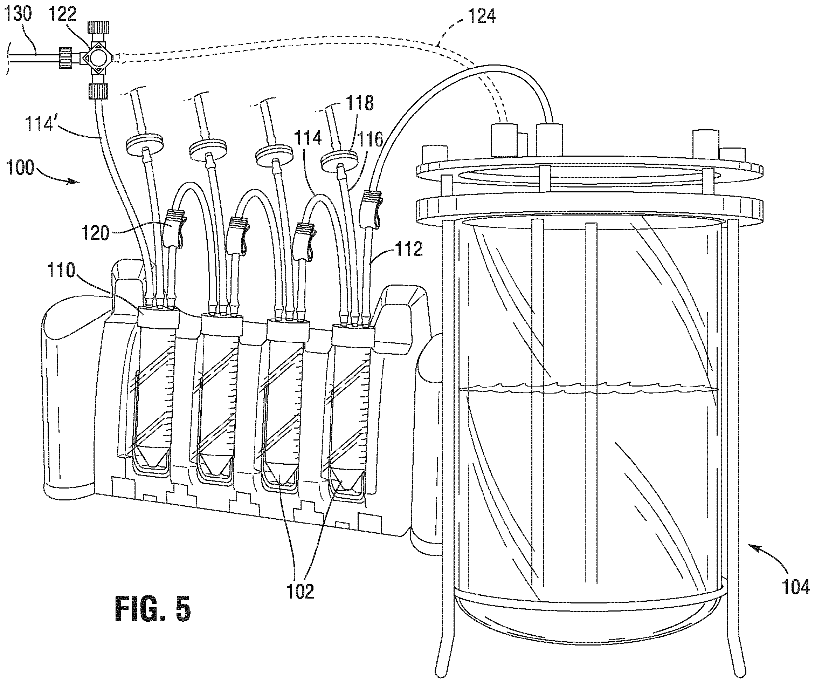

FIG. 5 illustrates a system 100 having multiple fluid receiving and sampling containers 102 connected in series and to a bioreactor 104. Each fluid receiving and sampling container 102 is configured as described above and has a lid held on by a cap 110. The lid includes three connectors to which are attached flexible tubes. In particular, as indicated for the container 102 closest to the bioreactor 104, there is an inlet tube 112, an outlet tube 114, and a vent tube 116. Each vent tube 116 may have a disc filter 118 therein, and optionally an on/off pinch valve (not shown) if venting is not desired or needed.

Each of the inlet tubes 112 has an on/off pinch valve 120 so that flow may be regulated between the bioreactor 104 and the series of containers 102, and between each container. The outlet tubes 114 connect to the inlet connector of the next container in series, until a final outlet tube 114' leads to a three-way stopcock 122. The stopcock 122 enables flow out of the last container 102 to be ported to different external locations, or even back to the bioreactor 104 as indicated in dashed line by an optional return line 124.

Connecting several closed-loop containers 102 in series in this manner permits several stages of growth and/or differentiation to occur in the relatively concentrated environment of the smaller volumes. The results may then be removed as end products via line 130, or channeled back into the bioreactor 104 for further processing. Additionally, though not shown, the containers 102 may be connected in parallel to the larger bioreactor 104 to enable more than one isolated reaction outside the larger vessel, and subsequent return to the vessel. The reactions in the containers 102 may be the same as or different than that taking place within the bioreactor 104, and the same as or different than each other. This can make the main reaction more effective or be used to change the direction of the reaction. To connect the containers 102 in parallel, the outlet tubes 114 of each would simply be routed back to the bioreactor 104.

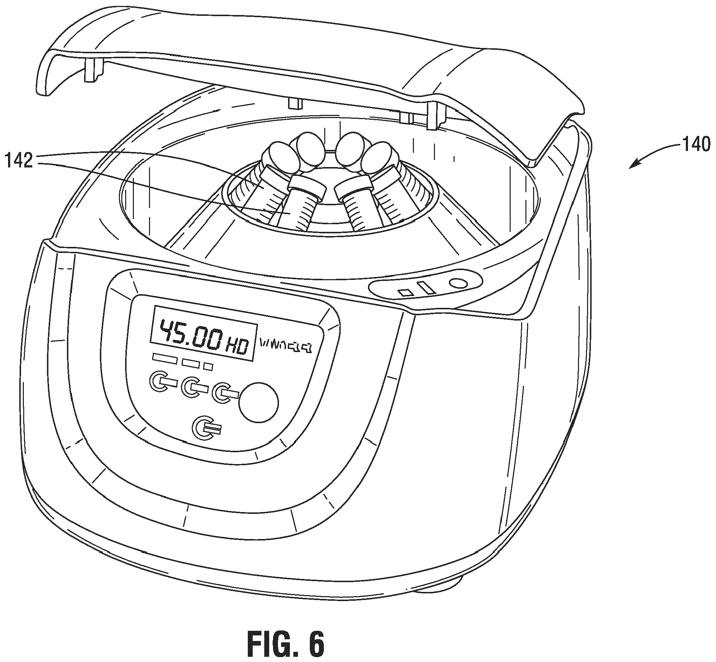

FIG. 6 is a schematic diagram of a centrifuge 140 for processing a plurality of fluid receiving and sampling containers 142 at once. This illustrates the potential for removing one or more containers 142 to be centrifuged to concentrate solid matter or other particles near the conical or dished bottom. As mentioned above, the receptacles have narrowed (conical, dished or rounded) or otherwise non-cylindrical or not flat closed bottom ends so that solids may be concentrated at the bottom in a centrifuge. The resulting "puck" or pellet of solid matter may be easily removed after withdrawing any remaining liquid via a pipette or by using the angled siphon end 62 on the outlet tube 60 as seen in FIG. 1B.

The exemplary closed transfer systems described above are useful in a number of areas aside from bead separation.

For instance, a filtration system can be provided and used for perfusion of the bioreactor and the addition of media/cell signals/specialized nutrients as cell engineering becomes more intricate. Spent media flows out of the reactor, cell signals (very small volume, hard to process due to tubing size and product loss within the necessary length of tubing) can be added to the filtering spent media allowing the use of a thinner, shorter line of tubing for the small volume of cell signal, or specialized nutrient. Cell differentiation processing or strategic media feeding are terms that encompass those areas for bioreactors.

In cellular engineering, cells are often concentrated down and reinoculated, sometimes in their same bioreactor. If it takes a while for the cell to expand, then new media is added for another cell expansion step before there is enough cell concentration for a larger bioreactor to be inoculated. The conical tube could be replaced with the cell separating device for this step bit the cell are intact and refed into the bioreactor.

The cell separation device could also be reconfigured to include a different filter, one which captures cell debris during AAV production, the cell is lysed to release the AAV for harvest and downstream processing. This is an area within the gene therapy space which has been difficult to achieve great yields. Right now, thermo has a resin, Poros AAVX, available in prepacked and affinity resin, which is leading may larger margins depending on the AAV stereotype.

The beads can be used to adhere Lenti Virus for harvest/purification, then washed for release. Essentially using a bind and release chromatography step immediately following the bioreactor. The terms: bind, elute, and flow through purification may be used to encompass all types of purification.

Terms such as top, bottom, left and right are used herein, though the fluid manifolds may be used in various positions such as upside down. Thus, some descriptive terms are used in relative terms and not absolute terms.

Throughout this description, the embodiments and examples shown should be considered as exemplars, rather than limitations on the apparatus and procedures disclosed or claimed. Although many of the examples presented herein involve specific combinations of method acts or system elements, it should be understood that those acts and those elements may be combined in other ways to accomplish the same objectives. Acts, elements and features discussed only in connection with one embodiment are not intended to be excluded from a similar role in other embodiments.

As used herein, "plurality" means two or more. As used herein, a "set" of items may include one or more of such items. Use of ordinal terms such as "first", "second", "third", etc., in the claims to modify a claim element does not by itself connote any priority, precedence, or order of one claim element over another or the temporal order in which acts of a method are performed, but are used merely as labels to distinguish one claim element having a certain name from another element having a same name (but for use of the ordinal term) to distinguish the claimelements.

According to an exemplary embodiment, the closed system for receiving and sampling liquid may comprise: a receptacle with solid walls and no openings defining a closed bottom end and an open top end, the closed bottom end being narrowed and not flat, the receptacle having an inner volume of 50 ml or less; a subassembly of a lid capable of sealing within and over the open top end of the receptacle, a first tube extending downward from the lid in fluid communication with a first connector projecting upward from the lid, a second tube extending downward from the lid in fluid communication with a second connector projecting upward from the lid, and a vent connector projecting upward from the lid and having an opening to a lower side of the lid, wherein when the lid is sealed over the top end of the receptacle the first, second and vent connectors provide exclusive avenues of ingress and egress to and from the inner volume and the first and second tubes extend downward into the inner volume of the receptacle with the second tube extending farther downward than the first tube; and a ring-shaped cap configured to be secured around the open top end of the receptacle so as to retain the lid over the open top end, wherein the closed system is manufactured and sold in a closed configuration with the first, second and vent connectors being sealed. The closed system of claim 1, wherein the first tube extends vertically downward and curves radially outward at a lower end, and is positioned to be spaced closely adjacent to an inner wall of the closed bottom end of the receptacle.

Disclosed herein is the above exemplary embodiment, wherein the lower end curves 90° or less and an end face of the lower end is angled perpendicular to the inner wall of the closed bottom end of the receptacle.

Disclosed herein is the above exemplary embodiment, wherein the second tube terminates within 0.05 inches of the closed bottom end of the receptacle.

Disclosed herein is the above exemplary embodiment, wherein the second tube has a small cut out across its longitudinal axis at its lower end to prevent the lower end from sealing against the closed bottom end of the receptacle from suction.

Disclosed herein is the above exemplary embodiment, wherein the receptacle narrows inward at the closed bottom end to a central location, and a lower end of the second tube angles so as to terminate in close proximity to the central location of the bottom end.

Disclosed herein is the above exemplary embodiment, wherein the subassembly is injection molded as one piece.

Disclosed herein is the above exemplary embodiment, further including a flexible first tube connected to the first connector and a first plug for sealing the flexible first tube, and a flexible second tube connected to the second connector and a second plug for sealing the flexible second tube.

Disclosed herein is the above exemplary embodiment, further including a vent filter connected to the vent connector.

Disclosed herein is the above exemplary embodiment, further including a flexible first tube connected to the first connector, and a flexible second tube connected to the second connector, and an on/off pinch valve on both the flexible first tube and the flexible second tube to enable manual opening and closure of flow therethrough.

Disclosed herein is the above exemplary embodiment, wherein the receptacle narrows inward at the closed bottom end to a central location, and wherein a lower end of the second tube angles so as to terminate in close proximity to the central location of the closed bottom end.

Disclosed herein is the above exemplary embodiment, wherein the first tube extends vertically downward and curves radially outward at a lower end, and is positioned to be spaced closely adjacent to an inner side wall of the receptacle.

Disclosed herein is the above exemplary embodiment, wherein the lower end of the first tube curves 90° or less so that an end face of the lower end is angled with respect to the inner side wall.

Disclosed herein is the above exemplary embodiment, wherein lower end of the second tube terminates within 0.05 inches of the closed bottom end of the receptacle.

Disclosed herein is the above exemplary embodiment, wherein the second tube has a small cut out across its longitudinal axis at its lower end to prevent the lower end from sealing against the closed bottom end of the receptacle from suction.

Disclosed herein is the above exemplary embodiment, wherein the subassembly is injection molded as one piece.

Disclosed herein is the above exemplary embodiment, further including a flexible first tube connected to the first connector and a first plug for sealing the flexible first tube, and a flexible second tube connected to the second connector and a second plug for sealing the flexible second tube.

Disclosed herein is the above exemplary embodiment, further including a three-way stopcock adapted to be coupled to the flexible second tube to enable distribution of the fluid from the second tube in multiple directions.

Disclosed herein is the above exemplary embodiment, further including an on/off pinch valve on both the flexible first tube and the flexible second tube to enable manual opening and closure of flow therethrough.

Disclosed herein is the above exemplary embodiment, further including a plug on the first connector, second connector and vent connectors to seal the connectors prior to use.

## Claims

1. A chemical or biological processing assembly including:
a closed system for receiving and sampling liquid, comprising:
i. a receptacle having a closed lower end and an open top end;
ii. a subassembly of a lid capable of sealing over the open top end of the receptacle, an inlet tube extending downward from the lid in communication with an inlet connector projecting upward from the lid, an outlet tube extending downward from the lid in communication with an outlet connector projecting upward from the lid, and a vent connector projecting upward from the lid and having an opening to a lower side of the lid, wherein the subassembly is configured to seal over the top end of the receptacle with the inlet tube and the outlet tube extending downward into an inner volume of the receptacle, and wherein the outlet tube extends farther downward than the inlet tube; and
iii. a cap ring configured to be secured around the open top end of the receptacle so as to retain the lid over the open top end; and
a reactor containing a liquid reaction medium with a reactor outlet and reactor outlet tube attached to the inlet connector of the subassembly, a stand having a plurality of stations adapted for holding the receptacle, the stand having magnets or is magnetized on an inner vertical wall at each of the stations, and wherein the receptacle contains magnetically-attractive beads configured to attract a desirable component of the liquid reaction medium, such that the desirable component may be isolated on the beads and held against an inner wall of the receptacle adjacent the inner vertical wall of a respective station.

2. The assembly of claim 1, wherein the inlet tube extends vertically downward and curves radially outward at a lower end, and is configured to be spaced closely adjacent to an inner wall of the receptacle at the lower end.

3. The assembly of claim 2, wherein the lower end curves 90° or less and an end face of the lower end is angled perpendicular so as to face the inner wall of the receptacle at the lower end.

4. The assembly of claim 1, wherein the outlet tube reaches to within 0.05 inches of a bottom end of the receptacle.

5. The assembly of claim 1, wherein the outlet tube has a small cut out across its longitudinal axis at its lower end to prevent the lower end from sealing against the bottom end of the receptacle from suction.

6. The assembly of claim 1, wherein the receptacle tapers inward at a bottom end, and a lower siphon end of the outlet tube angles so as to reach a central location of the bottom end.

7. The assembly of claim 1, wherein the subassembly is injection molded as one piece.

8. The assembly of claim 7, wherein the subassembly is made of polypropylene.

9. The assembly of claim 1, further including a flexible first tube connected to the inlet connector and a first plug for sealing the flexible first tube, and a flexible second tube connected to the outlet connector and a second plug for sealing the flexible second tube.

10. The assembly of claim 9, further including a three-way stopcock adapted to be coupled to the flexible second tube to enable distribution of the fluid from the outlet tube in multiple directions.

11. The assembly of claim 1, further including a vent filter connected to the vent connector.

12. The assembly of claim 1, further including a plug on both the inlet tube and the outlet tube to seal the tubes prior to use.

13. The assembly of claim 1, further including a flexible first tube connected to the inlet connector, and a flexible second tube connected to the outlet connector, and an on/off pinch valve on both the flexible first tube and the outlet flexible second tube to enable manual opening and closure of flow therethrough.

14. The assembly of claim 1, wherein there are a plurality of the closed systems placed in the plurality of stations of the stand each of which is connected to receive liquid reaction medium from the reactor.

15. The assembly of claim 1, further including a syringe attached to the outlet connector of the closed system for sampling fluid from within the receptacle.

16. A chemical or biological processing assembly including:
a closed system for receiving and sampling liquid, comprising:
i. a receptacle having a closed lower end and an open top end;
ii. a subassembly of a lid capable of sealing over the open top end of the receptacle, an inlet tube extending downward from the lid in communication with an inlet connector projecting upward from the lid, an outlet tube extending downward from the lid in communication with an outlet connector projecting upward from the lid, and a vent connector projecting upward from the lid and having an opening to a lower side of the lid, wherein the subassembly is configured to seal over the top end of the receptacle with the inlet tube and the outlet tube extending downward into an inner volume of the receptacle, and wherein the outlet tube extends farther downward than the inlet tube; and
iii. a cap ring configured to be secured around the open top end of the receptacle so as to retain the lid over the open top end; and
a reactor containing a liquid reaction medium with a reactor outlet and reactor outlet tube attached to the inlet connector of the subassembly, and a three-way stopcock attached to the outlet connector of the closed system, a return tube connected to a junction of the three-way stopcock and also to a return inlet to the reactor, and a sampling tube connected to a junction of the three-way stopcock.

17. The assembly of claim 16, wherein the inlet tube extends vertically downward and curves radially outward at a lower end, and is configured to be spaced closely adjacent to an inner wall of the receptacle at the lower end.

18. The assembly of claim 17, wherein the lower end curves 90° or less and an end face of the lower end is angled perpendicular so as to face the inner wall of the receptacle at the lower end.

19. The assembly of claim 16, wherein the outlet tube reaches to within 0.05 inches of a bottom end of the receptacle.

20. The assembly of claim 16, wherein the outlet tube has a small cut out across its longitudinal axis at its lower end to prevent the lower end from sealing against the bottom end of the receptacle from suction.

21. The assembly of claim 16, wherein the receptacle tapers inward at a bottom end, and a lower siphon end of the outlet tube angles so as to reach a central location of the bottom end.

22. The assembly of claim 16, wherein the subassembly is injection molded as one piece.

23. The assembly of claim 22, wherein the subassembly is made of polypropylene.

24. The assembly of claim 16, further including a flexible first tube connected to the inlet connector and a first plug for sealing the flexible first tube, and a flexible second tube connected to the outlet connector and a second plug for sealing the flexible second tube.

25. The assembly of claim 16, further including a vent filter connected to the vent connector.

26. The assembly of claim 16, further including a plug on both the inlet tube and the outlet tube to seal the tubes prior to use.

27. The assembly of claim 16, further including a flexible first tube connected to the inlet connector, and a flexible second tube connected to the outlet connector, and an on/off pinch valve on both the flexible first tube and the flexible second tube to enable manual opening and closure of flow therethrough.
